# EUROPEAN PATENT SPECIFICATION

(11) **EP 0 714 400 B1**
(45) Date of publication and mention of the grant of the patent: **15.12.1999**
(21) Application number: 94926502.9
(22) Date of filing: 15.08.1994
(51) Int. Cl.: C07H 17/08, A01N 43/90

(54) **THERMODYNAMICALLY STABLE CRYSTAL FORM OF 4"-DEOXY-4"-(EPI)-METHYLAMINO AVERMECTIN B1a/B1b BENZOIC ACID SALT AND PROCESSES FOR ITS PREPARATION**
THERMODYNAMISCH STABILE KRISTALLFORM VON 4"-DEOXY-4"-EPI-METHYLAMINO AVERMECTIN BIA/BIB BENZOESAURESALZ UND VERFAHREN ZU SEINER HERSTELLUNG
FORME CRISTALLINE THERMODYNAMIQUEMENT STABLE D'UN SEL D'ACIDE BENZO QUE DE 4"-DESOXY-4"-(EPI)-METHYLAMINO AVERMECTINE B1a/B1b ET PROCEDES DE PREPARATION DUDIT SEL

(30) Priority: 19.08.1993 US 109189
(43) Date of publication of application: 05.06.1996
(73) Proprietor: Merck & Co., Inc., Rahway New Jersey 07065-0900 (US)
(72) Inventor: CVETOVICH, Raymond, Scotch Plains, NJ 07076 (US); DEMCHAK, Richard, Langhorne, PA 19047 (US); McCAULEY, James, A., Belle Mead, NJ 08502 (US); VARSALONA, Richard, J., Scotch Plains, NJ 07076 (US)
(74) Representative: Hiscock, Ian James
(86) International application number: US9409207
(87) International publication number: WO9505390

(56) References cited:
- EP-A- 0 465 121

## Description

### BACKGROUND OF THE INVENTION

The benzoic acid salt of 4"-deoxy-4"-epi-methylamino avermectin B1a/B1b is known and known to be a stable salt as described in European Patent Publication EP 0465121-A1. The added stability relative to the stability of the previously described hydrochloride salt provided much greater shelf life for this important agricultural insecticide.

### SUMMARY OF THE INVENTION

This invention is concerned with the most stable of four crystalline forms of the benzoic acid salt of 4"-deoxy-4"-epi-methyl-amino avermectin B1a/B1b a known agricultural insecticide, and processes for preparing it.

The four crystalline forms that have been identified are designated as forms A, B, C and D, three of which are hydrated (B, C and D). The most thermodynamically stable crystalline form is the hemi-hydrate B. Knowing the most stable crystalline form and devising processes for producing the product in that form is extremely important in that it provides bulk material with crystal homogeneity that is not going to transform to another crystal habit on storage.

The novel processes for producing crystal form B comprises crystallization of the compound from an organic solvent containing a controlled amount of water.

### DETAILED DESCRIPTION OF THE INVENTION

The novel compound of this invention is crystalline 4"-deoxy-4"-epi-methylamino avermectin B1a/B1b, benzoic acid salt, hemihydrate in substantially pure form.

It has structural formula: 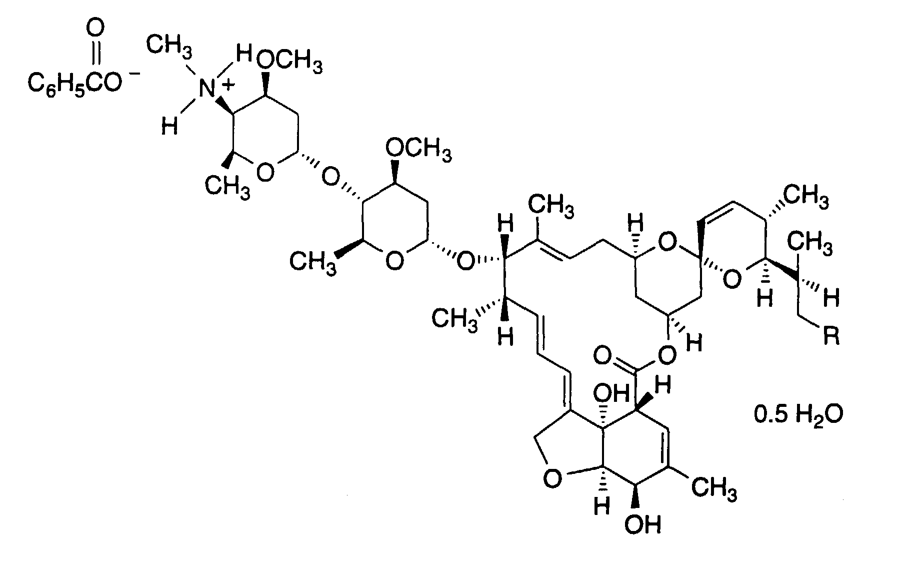 wherein R is -CH₃ (B1a) or -H (B1b) and is referred to as Crystal form B, Type B or similar designation.

The differential scanning calorimetry (DSC) curve determined at a heating rate of 20°C/min. under nitrogen flow in an open cup is characterized by a relatively broad water loss endotherm with a peak temperature of 74°C and a major melting/decomposition endotherm with peak temperature of 155°C, extrapolated onset temperature of 150°C with an associated heat of 56 Joules/gm. The x-ray powder diffraction pattern for Type B is characterized by d-spacings of 18.13, 9.08, 8.68, 5.03, 4.61, 4.53, 3.97 and 3.82Å. The aqueous solubility is pH dependent. At pH 5, acetate buffer, it has a solubility of 0.32 mg/ml.

The novel process of this invention comprises recrystallizing the compound in any energetic state from an aqueous organic solvent.

The aqueous organic solvent useful in the novel process is preferably acetonitrile with 2-4% w/w of water; methyl t-butyl ether (MTBE) with 0.5 to 0.8% w/w of water; or isopropanol (IPA) with 0.1-0.3% w/w of water.

Compound of crystal form A, C or D is dissolved in the organic solvent at about 50-60°C treated with the requisite amount of water, seeded with Type B crystals, cooled to and aged at about 15-30°C for about 2-4 hours and further cooled to about 5°C over a period of about 1-2 hours.

The crystalline product is collected on a filter and dried in vacuo.

The utility and methods of use of the benzoic acid salt described herein and the free base thereof are well known by those skilled in the art and fully described in the scientific and patent literature such as EP 0465121.

It has significant parasiticidal activity as an anthelmintic, ectoparasiticide, insecticide and acaricide, in human and animal health and in agriculture. As an agricultural pesticide it has activity against insect pests of stored grains such as Tribolium sp., Tenebrio sp., and of agricultural plants such as spider mites, (Tetranychus sp.), aphids, (Acyrthiosiphon sp.); against migratory orthopterans such as locusts and immature stages of insects living on plant tissue. The compound is useful as a nematocide for the control of soil nematodes and plant parasites such as Meloidogyne sp. which is of importance in agriculture. The compound is active against other plant pests such as the southern army worm and Mexican bean beetle larvae.

The compounds are applied using known techniques as sprays, dusts, emulsions and the like, to the growing or stored crops to effect protection from such agricultural pests. For the treatment of growing crops, the compound is administered at a rate of about 5-50 gms per hectare. For the protection of stored crops it is normally administered by spraying with a solution containing from 0.1-10 ppm. of the compound.

### EXAMPLE

| Preparation of Type B Product | |
|---|---|
| Materials | Amounts |
| | 3.59 Kg 94.2 wt% |
| TYPE A MATERIAL (obtained by crystallization from MTBE) | 2.33 Kg 95.7 wt% |
| | 5.92 Kg (5.61 Kg by assay) |
| | |
| ACETONITRILE (HPLC GRADE) | 40 L (KF = 0.2 mg/ml) |
| | |
| WATER (DI) | 570 mL |

### Procedure

A 50 L 4-neck round bottom flask was equipped with a mechanical stirrer, N₂ inlet, thermocouple and funnel. After purging the vessel with N₂, acetonitrile (12 L, KF = 0.2 mg/ml) was added and warmed to 50-55°C, and Type A material was charged. Upon complete addition (30 min.), the mixture was aged an additional 10 min. in a nitrogen atmosphere to give a clear red-brown solution.

The batch was diluted with additional acetonitrile (6 L), water (270 mL) was added, and then seed crystals of form Type B (10 g) were added at 45°C. Crystal growth began immediately and rapidly at 45°C. Crystallization must initiate, and largely occur at >35°C to assure formation of Type B crystals.

The batch was cooled to 20°C over a 45 min period and aged for 2.5 hrs. The crystalline slurry thickened during this period and made stirring difficult. Additional acetonitrile (2L) was added, and the slurry was cooled to 5°C over 1 hr. After aging at 5°C for 30 min, the slurry was filtered onto a 22-in. diameter Cenco-Lapp funnel lined with paper and polypropylene. The mother liquor was recycled back to the crystallization vessel to rinse and transfer the remaining product. The cake was washed with cold (2°C) wet acetonitrile (5 X 4L, each 1.5% (v/v) in water). The cake was partially dried with a stream of nitrogen passed through the cake for 30 min, and it was then transferred to the Hull dryer.

The cake was dried at 30°C in vacuo (25 in.) over 3 days to a constant weight and an acetonitrile content <0.1 wt% by GC. The batch product was 4.95 Kg of Type B material.

| Analyses B1a anhydrous | |
|---|---|
| MW | 1008.26 |
| FORMULA | C₅₆H₈₁NO₁₅ |
| CFA: | OFF-WHITE POWDER |
| X-RAY | CRYST. TYPE B HYDRATE |
| HPLC* | 95.9 WT% |
| | 97.8 area% |
| TLC | SINGLE SPOT |
| TITRATION* | 100% (Perchloric acid) |
| UV* | A% 333,245 nm in methanol |
| KF | 1.08 wt% |
| GC | CH₃CN <0.01% |
| | HEXANES <0.01% |
| | MTBE nd, <0.01% |
| | IPAc nd, <0.01% |

| | |
|---|---|
| *corrected for KF, GC | |

## Claims

1. A crystalline compound 4"-deoxy-4"-*epi*-methyl-amino avermectin B1a/B1b, benzoic acid salt, hemihydrate in substantially pure form.

2. A process for the preparation of the crystalline compound 4"-deoxy-4"-*epi*-methylamino avermectin B1a/B1b, benzoic acid salt, hemihydrate (Type B) in substantially pure form which comprises recrystallizing any other form or mixtures of forms thereof from an aqueous organic solvent by dissolving the starting material in the organic solvent at a temperature of 50-60°C adding about 0.1 to 4% w/w of water; cooling to about 45-35°C; seeding with Type B crystals; holding until crystallization is essentially complete; cooling to and holding at 15 to 30°C for about 2-4 hours, cooling to about 5°C over a period of 1-2 hours, collecting the solids and drying.

3. The process of Claim 2 wherein the aqueous organic solvent is acetonitrile with 2-4% w/w of water; methyl t-butyl ether with 0.5 to 0.8% w/w of water; or isopropanol with 0.1 to 0.3% w/w of water.

4. The process of Claim 3 wherein the aqueous solvent is acetonitrile with 2-4% w/w of water.

5. A method for the control of agricultural insects, which comprises applying to an area infested with such agricultural insects an effective amount of the stable salt of Claim 1.

6. A composition useful for the treatment of insect infestations of plants or plant products which comprises an inert carrier and an effective amount of the stable salt of Claim 1.

## Patentansprüche

1. Eine kristalline Verbindung 4''-Desoxy-4''-epi-methylaminoavermectin B1a/B1b-Benzoesäuresalz-Hemihydrat in im wesentlichen reiner Form.

2. Ein Verfahren für die Herstellung der kristallinen Verbindung 4''-Desoxy-4''-epi-methylaminoavermectin B1a/B1b-Benzoesäuresalz-Hemihydrat (Typ B) in im wesentlichen reiner Form, das Umkristallisieren irgendeiner anderen Form oder von Hischungen von Formen davon aus einem wäßrigen organischen Lösungsmittel durch Auflösen des Ausgangsmaterials in dem organischen Lösungsmittel bei einer Temperatur von 50-60°C, Zugabe von etwa 0,1 bis 4% Gew./Gew. Wasser, Abkühlen auf etwa 45-35°C, Impfen mit Typ-B-Kristallen, Abwarten, bis die Kristallisation im wesentlichen vollständig ist, Abkühlen auf und Abwarten bei 15 bis 30°C für etwa 2-4 Stunden, Abkühlen auf etwa 5°C über einen Zeitraum von 1-2 Stunden, Sammeln der Feststoffe und Trocknen umfaßt.

3. Das verfahren nach Anspruch 2, wobei das wäßrige organische Lösungsmittel Acetonitril mit 2-4% Gew./Gew. Wasser, Methyl-t-butylether mit 0,5 bis 0,8% Gew./Gew. Wasser oder Isopropanol mit 0,1 bis 0,3% Gew./Gew. Wasser ist.

4. Das Verfahren nach Anspruch 3, wobei das wäßrige Lösungsmittel Acetonitril mit 2-4% Gew./Gew. Wasser ist.

5. Ein Verfahren zur Bekämpfung von Agrikultur-Insekten, das das Ausbringen einer wirksamen Menge des stabilen Salzes nach Anspruch 1 auf eine mit solchen Agrikultur-Insekten befallene Fläche umfaßt.

6. Eine Zusammensetzung, die für die Behandlung von Insektenbefall von Pflanzen oder Pflanzenprodukten nützlich ist und die einen inerten Träger und eine wirksame Menge des stabilen Salzes nach Anspruch 1 enthält.

## Revendications

1. Composé cristallin qui est le sel d'acide benzoïque de la 4"-désoxy-4"-épi-méthylaminoavermectine B1a/B1b hémihydraté, sous forme essentiellement pure.

2. Procédé de préparation du composé cristallin qu'est le sel d'acide benzoïque de la 4"-désoxy-4"-épi-méthylaminoavermectine B1a/B1b hémihydraté (type B) sous forme essentiellement pure, qui comprend la recristallisation d'une autre forme quelconque ou de mélanges de ces formes dans un solvant organique par dissolution du produit de départ dans le solvant organique à une température de 50-60°C, avec addition d'environ 0,1 à 4% en poids/poids d'eau, refroidissement à environ 45-35°C; ensemencement avec des cristaux de type B, maintien jusqu'à ce que la cristallisation soit essentiellement terminée, refroidissement et maintien à 15-30°C pendant environ 2-4 heures, refroidissement à environ 5°C en l'espace de 1-2 heures, récupération des matières solides et séchage.

3. Procédé selon la revendication 2, dans lequel le solvant organique aqueux est l'acétonitrile avec 2-4% en poids/poids d'eau; le méthyl-t-butyléther avec 0,5 à 0,8% en poids/poids d'eau; ou l'isopropanol avec 0,1 à 0,3% en poids/poids d'eau.

4. Procédé selon la revendication 3, dans lequel le solvant aqueux est l'acétonitrile avec 2-4% en poids/poids d'eau.

5. Procédé pour lutter contre des insectes agricoles, qui comprend l'application sur une zone infestée par ces insectes agricoles d'une quantité efficace du sel stable de la revendication 1.

6. Composition utile pour le traitement d'infestations d'insectes sur des végétaux ou des produits végétaux, qui comprend un véhicule inerte et une quantité efficace du sel stable de la revendication 1.
